# EUROPEAN PATENT APPLICATION

(11) **EP 3 851 103 A1**
(43) Date of publication of application: **21.07.2021**
(21) Application number: 19860814.3
(22) Date of filing: 10.09.2019
(51) Int. Cl.: A61K 31/415, A61P 13/12

(54) **THERAPEUTIC OR PROPHYLACTIC AGENT FOR CHRONIC KIDNEY DISEASE CONTAINING PYRAZOLE-AMIDE COMPOUND**

(30) Priority: 11.09.2018 JP 2018169632
(71) Applicant: Japan Tobacco Inc., Tokyo 105-6927 (JP)
(72) Inventor: SAITO, Tomoyuki, Takatsuki-shi, Osaka 569-1125 (JP); OHTA, Takeshi, Takatsuki-shi, Osaka 569-1125 (JP); KAKUTANI, Makoto, Takatsuki-shi, Osaka 569-1125 (JP); MERA, Yasuko, Takatsuki-shi, Osaka 569-1125 (JP)
(74) Representative: Vossius & Partner Patentanwälte Rechtsanwälte mbB
(86) International application number: PCT/JP2019/035586
(87) International publication number: WO 2020/054734

(57) **Abstract**

The present invention aims to provide novel pharmaceutical use of compound A or a pharmaceutically acceptable salt thereof, or a monohydrate of compound A. The present invention relates to a therapeutic or prophylactic agent for chronic kidney diseases containing compound A or a pharmaceutically acceptable salt thereof, or a monohydrate of compound A as the active ingredient.

## Description

### [Technical Field]

The present invention relates to novel pharmaceutical use of 2-{4-[(9R)-9-hydroxy-2-(3-hydroxy-3-methylbutyloxy)-9-(trifluoromethyl)-9H-fluoren-4-yl]-1H-pyrazol-1-yl}-2-methylpropanamide (hereinafter indicated as compound A) or a pharmaceutically acceptable salt thereof, or a monohydrate of compound A. More particularly, the present invention relates to a therapeutic or prophylactic agent for chronic kidney diseases containing compound A or a pharmaceutically acceptable salt thereof, or a monohydrate of compound A.

### [Background Art]

Chronic kidney disease (CKD) is a disease in which renal disorder and deterioration of renal function are sustained. Progression of CKD leads to an end-stage renal disease (ESRD) for which dialysis and kidney transplantation are necessary. Many clinical studies have shown that CKD increases the risk of cardiovascular diseases such as myocardial infarction, stroke, heart failure and the like, as well as death. Therefore, it is important to prevent the aggravation of CKD and suppress the onset of cardiovascular diseases.

CKD is defined as a condition in which renal disorder and/or reduced glomerular filtration rate (GFR) (less than 60 mL/min/1.73 m²) continues for three months or longer. Renal disorder is diagnosed by urine abnormality, image diagnosis, blood, and pathology, and the presence of protein urine (protein urine of 0.15 g/gCr or more or albumin urine of 30 mg/gCr or more) is particularly important in urine abnormality. GFR is measured by inulin clearance, and is also simply evaluated by estimated GFR (eGFR) using serum creatinine values.

CKD shows almost no subjective symptoms at the initial stage, but as it progresses, symptoms such as nocturia, edema, anemia, fatigue, shortness of breath and the like are observed.

As animal models exhibiting CKD-like renal disorder and decreased renal function, salt-loaded SDT-fatty rats, 5/6 nephrectomized rats, and the like are known (non-patent documents 1, 2).

Compound A, 2-{4-[(9R)-9-hydroxy-2-(3-hydroxy-3-methylbutyloxy)-9-(trifluoromethyl)-9H-fluoren-4-yl]-1H-pyrazol-1-yl}-2-methylpropanamide, a pharmaceutically acceptable salt thereof, and a monohydrate of compound A are described in patent document 1 and patent document 2. Patent document 1 describes that compound A and a monohydrate thereof have a pyruvate dehydrogenase kinase (PDHK) inhibitory activity and may become medicaments effective for diabetes, insulin resistance syndrome, metabolic syndrome, hyperglycemia, hyperlactacidemia, diabetic complications, cardiac failure, cardiomyopathy, myocardial ischemia, myocardial infarction, angina pectoris, dyslipidemia, atherosclerosis, peripheral arterial disease, intermittent claudication, chronic obstructive pulmonary diseases, brain ischemia, stroke, mitochondrial disease, mitochondrial encephalomyopathy, cancer or pulmonary hypertension. Patent document 2 describes a production method of compound A or a pharmaceutically acceptable salt thereof, or a monohydrate of compound A.

### [Document List]

### [Patent documents]

patent document 1: WO 2014/142290
patent document 2: WO 2018/021508

### [Non-patent documents]

non-patent document 1: KATSUDA, Yoshiaki, et al. Physiological changes induced by salt intake in female Spontaneously Diabetic Torii-Lepr(fa) (SDT fatty) rat, a novel obese type 2 diabetic model. Animal science journal, 2014, 85(5), 588-594. non-patent document 2: TSUPRYKOV, Oleg, et al. The dipeptidyl peptidase inhibitor linagliptin and the angiotensin II receptor blocker telmisartan show renal benefit by different pathways in rats with 5/6 nephrectomy. Kidney international, 2016, 89(5), 1049-1061.

### [Summary of Invention]

### [Technical Problem]

The problem to be solved by the present invention is to provide a therapeutic drug for CKD.

### [Solution to Problem]

The present inventors have found in experiments using disease model animals exhibiting CKD-like renal disorder and decreased renal function that compound A or a pharmaceutically acceptable salt thereof, or a monohydrate of compound A significantly reduces the severity of the disease. Based on this finding, the present inventors have found that compound A or a pharmaceutically acceptable salt thereof, or a monohydrate of compound A can be an effective medicament for CKD, and completed the present invention.

That is, the present invention provides the following.
[1] A therapeutic or prophylactic agent for a chronic kidney disease, comprising a compound represented by the following chemical structural formula: 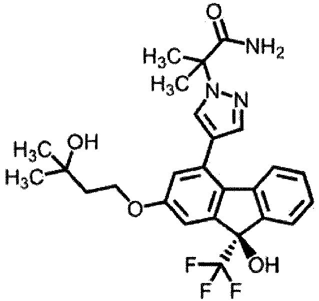 or a pharmaceutically acceptable salt thereof, or a compound represented by the following chemical structural formula: 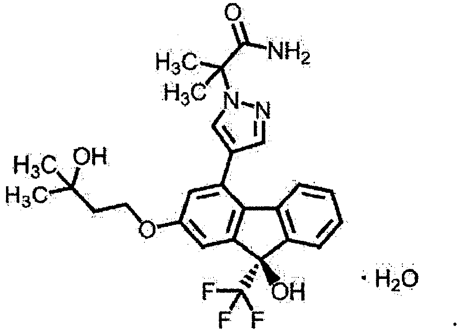
[2] A renal function improving agent comprising a compound represented by the following chemical structural formula: 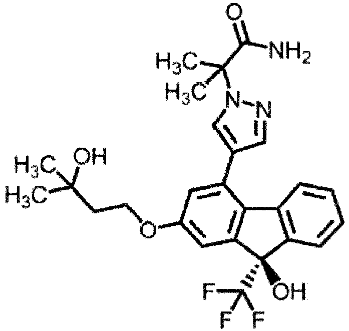 or a pharmaceutically acceptable salt thereof, or a compound represented by the following chemical structural formula: 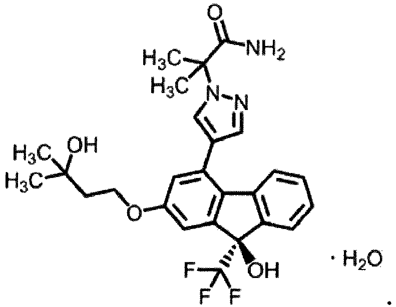
[3] An agent for improving one or more parameters selected from GFR, eGFR, serum creatinine value, blood urea nitrogen value, creatinine clearance, urinary protein value and urinary albumin value, comprising a compound represented by the following chemical structural formula: 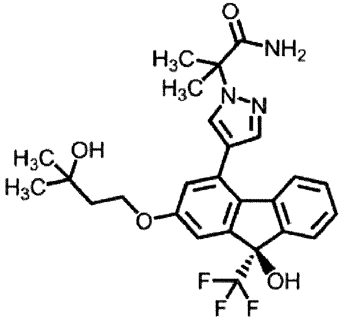 or a pharmaceutically acceptable salt thereof, or a compound represented by the following chemical structural formula: 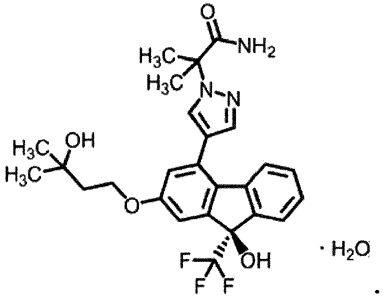
[4] An agent for improving GFR, comprising a compound represented by the following chemical structural formula: 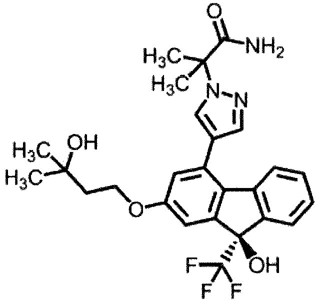 or a pharmaceutically acceptable salt thereof, or a compound represented by the following chemical structural formula: 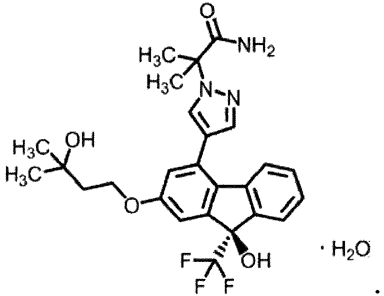
[5] An agent for improving eGFR, comprising a compound represented by the following chemical structural formula: 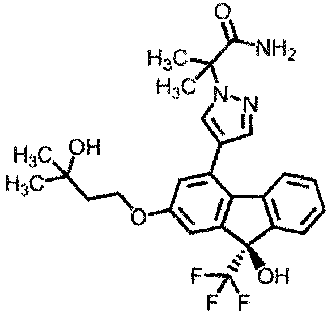 or a pharmaceutically acceptable salt thereof, or a compound represented by the following chemical structural formula: 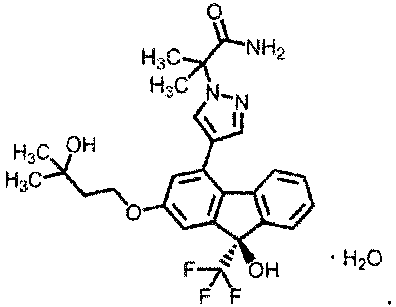
[6] An agent for improving a serum creatinine value, comprising a compound represented by the following chemical structural formula: 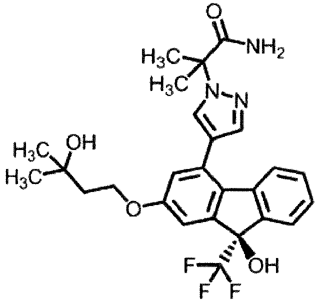 or a pharmaceutically acceptable salt thereof, or a compound represented by the following chemical structural formula: 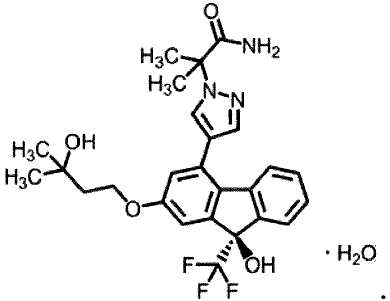
[7] An agent for improving a blood urea nitrogen value, comprising a compound represented by the following chemical structural formula: 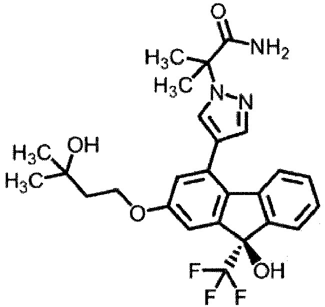 or a pharmaceutically acceptable salt thereof, or a compound represented by the following chemical structural formula: 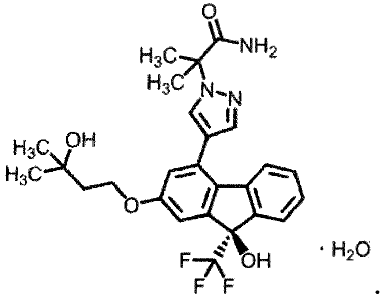
[8] An agent for improving creatinine clearance, comprising a compound represented by the following chemical structural formula: 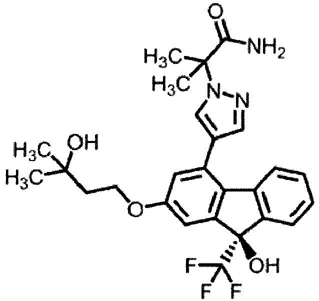 or a pharmaceutically acceptable salt thereof, or a compound represented by the following chemical structural formula: 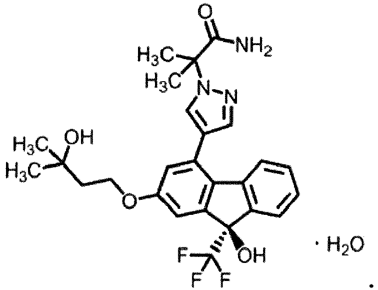
[9] An agent for improving a urinary protein value, comprising a compound represented by the following chemical structural formula: 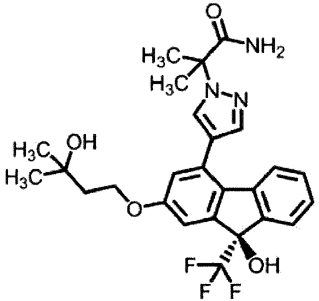 or a pharmaceutically acceptable salt thereof, or a compound represented by the following chemical structural formula: 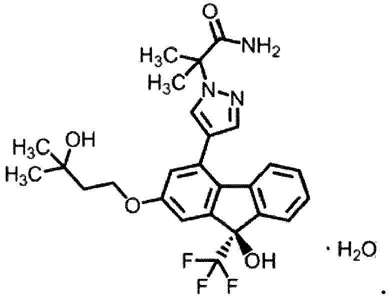
[10] An agent for improving a urinary albumin value, comprising a compound represented by the following chemical structural formula: 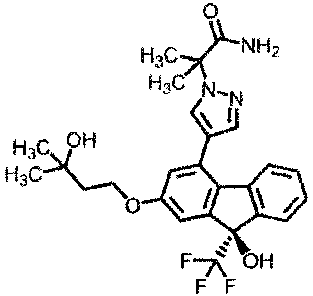 or a pharmaceutically acceptable salt thereof, or a compound represented by the following chemical structural formula: 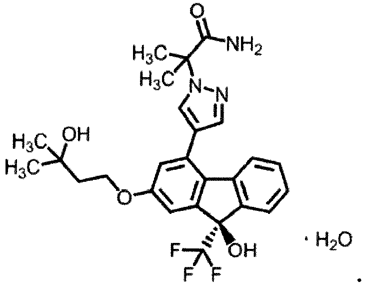
[11] The agent of any of the aforementioned [1] to [10], comprising a compound represented by the following chemical structural formula: 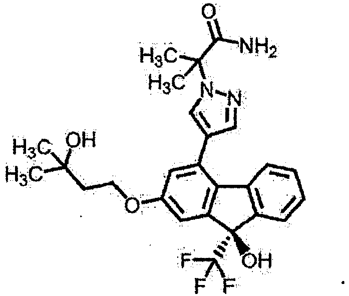
[12] The agent of any of the aforementioned [1] to [10], comprising a compound represented by the following chemical structural formula: 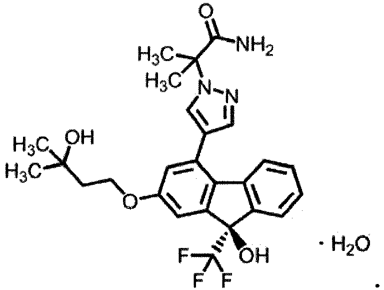
[13] The agent of the aforementioned [1], wherein the chronic kidney disease is a chronic kidney disease excluding diabetic nephropathy.
[14] The agent of the aforementioned [1], wherein the chronic kidney disease is a chronic kidney disease excluding a chronic kidney disease primarily caused by diabetes.
[15] A method for the prophylaxis or treatment of a chronic kidney disease in a mammal, comprising administering an effective amount of a compound represented by the following chemical structural formula: 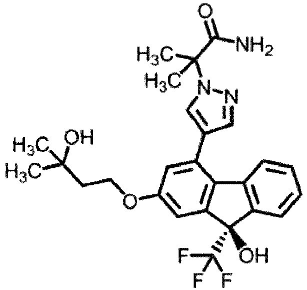 or a pharmaceutically acceptable salt thereof, or a compound represented by the following chemical structural formula: 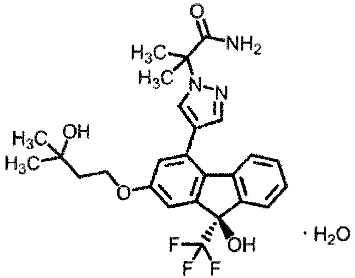 to the mammal.
[16] A compound represented by the following chemical structural formula: 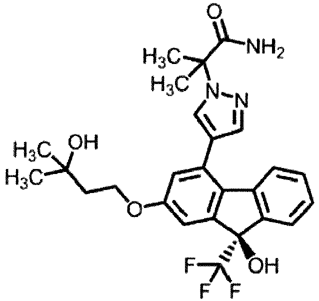 or a pharmaceutically acceptable salt thereof, or a compound represented by the following chemical structural formula: 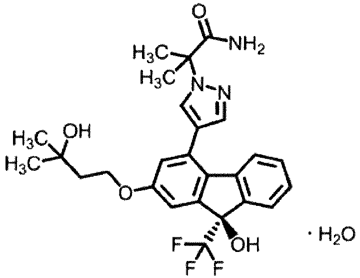 for use in the prophylaxis or treatment of a chronic kidney disease.
[17] Use of a compound represented by the following chemical structural formula: 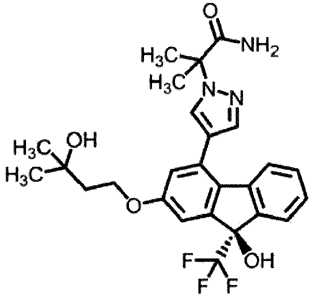 or a pharmaceutically acceptable salt thereof, or a compound represented by the following chemical structural formula: 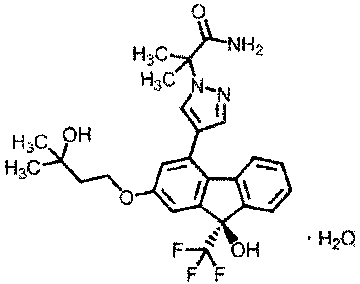 in producing a therapeutic or prophylactic agent for a chronic kidney disease.

### [Advantageous Effects of Invention]

The compound A or a pharmaceutically acceptable salt thereof, or a monohydrate of compound A in the present invention is effective as a therapeutic or prophylactic agent for CKD.

### [Brief Description of Drawings]

Fig. 1 shows amount of urinary protein excretion of saline-loaded SDT-fatty rats orally administered with water (Normal), saline (Vehicle), or saline and a monohydrate of compound A (compound A).
Fig. 2 shows representative examples of kidney tissue images of saline-loaded SDT-fatty rats each orally administered with (a) water, (b) saline, or (c) saline and a monohydrate of compound A.
Fig. 3 shows amount of urinary protein excretion of a 5/6 nephrectomized rat (Vehicle), a 5/6 nephrectomized rat orally administered with a monohydrate of compound A (compound A), and a rat without nephrectomy (Sham).

### [Description of Embodiments]

The definitions of the terms in the present specification are as follows.

The compound A is 2-{4-[(9R)-9-hydroxy-2-(3-hydroxy-3-methylbutyloxy)-9- (trifluoromethyl)-9H-fluoren-4-yl]-1H-pyrazol-1-yl}-2-methylpropanamide, and is represented by the following chemical structural formula:

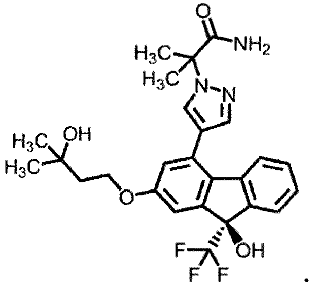

The "pharmaceutically acceptable salt" may be any salt known in the art that does not accompany excessive toxicity. Specifically, salts with inorganic acids, salts with organic acid, salts with inorganic bases, salts with organic bases and the like can be mentioned. Various forms of pharmaceutically acceptable salts are well known in the art and are described, for example, in the following reference documents:
(a) Berge et al., J. Pharm. Sci., 66, p1-19(1977),
(b) Stahl et al., "Handbook of Pharmaceutical Salts: Properties, Selection, and Use" (Wiley-VCH, Weinheim, Germany, 2002),
(c) Paulekuhn et al., J. Med. Chem., 50, p6665-6672 (2007).

A pharmaceutically acceptable salt of compound A can be each obtained by reacting compound A with an inorganic acid, an organic acid, an inorganic base or an organic base according to a method known per se. A pharmaceutically acceptable salt of compound A may be formed as a half molecule, one molecule, or two or more molecules of acid or base with respect to one molecule of compound A.

Examples of the salt with inorganic acid include salts with hydrofluoric acid, hydrochloric acid, hydrobromic acid, hydroiodic acid, nitric acid, phosphoric acid and sulfuric acid.

Examples of the salt with organic acid include salts with acetic acid, adipic acid, alginic acid, 4-aminosalicylic acid, anhydromethylenecitric acid, benzoic acid, benzenesulfonic acid, edetic acid calcium, camphoric acid, 10-camphorsulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glycolylarsanilic acid, hexylresorcinic acid, hydroxy-naphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis (salicylic acid), galactaric acid, naphthalene-2-sulfonic acid, 2-naphthoic acid, 1,5-naphthalene disulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid and glutamic acid.

Examples of the salt with inorganic base include salts with lithium, sodium, potassium, magnesium, calcium, barium, aluminum, zinc, bismuth and ammonium.

Examples of the salt with organic base include salts with arecoline, betaine, choline, clemizole, ethylenediamine, N-methylglucamine, N-benzylphenethylamine, tris(hydroxymethyl)methylamine, arginine and lysine.

The compound A or a pharmaceutically acceptable salt thereof, or a monohydrate of compound A can be produced by a known method, for example, the method described in patent document 1 or patent document 2.

The compound A or a pharmaceutically acceptable salt thereof may exist as a solvate. The term "solvate" refers to compound A or a pharmaceutically acceptable salt thereof with which a solvent molecule is coordinated, and also includes hydrates. Such solvates are preferably pharmaceutically acceptable solvates. Such solvates include, for example, hydrate, ethanol solvate, dimethylsulfoxide-solvate and the like of compound A or a pharmaceutically acceptable salt thereof. Specific examples include hemihydrate, monohydrate, dihydrate or mono(ethanol)solvate of compound A or a monohydrate of compound A, 2/3(ethanol)solvate of dihydrochloride of the same and the like. Such solvates can be produced according to conventional methods.

The solvate is preferably a hydrate of compound A, more preferably a monohydrate of compound A, and is represented by the following structural formula:

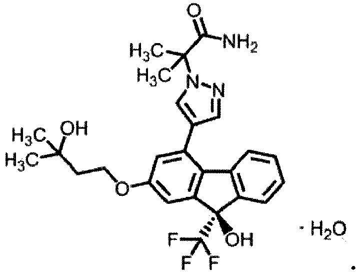

The therapeutic or prophylactic agent for CKD of the present invention is produced according to a method known in the art of pharmaceutical preparations, by mixing compound A or a pharmaceutically acceptable salt thereof, or a monohydrate of compound A with a suitable amount of at least one kind of pharmaceutically acceptable carrier and the like as appropriate. While the content of compound A or a pharmaceutically acceptable salt thereof, or a monohydrate of compound A in the preparation varies depending on the dosage form, dose and the like, it is, for example, 0.1 to 100 wt% of the whole agent.

The therapeutic or prophylactic agent of the present invention can be administered orally or parenterally. Examples of the administration form include oral administration and parenteral administration such as intravenous, intramuscular, subcutaneous, transdermal, topical, rectal administrations and the like. Examples of the dosage form suitable for oral administration include tablet, capsule, granule, powder, troche, syrup, emulsion, suspension and the like, and examples of the dosage form suitable for parenteral administration include external preparation, suppository, injection, eye drop, eye ointment, plaster, gel, insertion agent, nasal preparation, pulmonary preparation and the like. These can be prepared according to a method known in the field of pharmaceutical preparations.

Examples of the "pharmaceutically acceptable carrier" include various organic or inorganic carrier substances conventionally used as preparation materials, for example, excipient, disintegrant, binder, fluidizer, lubricant and the like for solid preparations, solvent, solubilizing agent, suspending agent, isotonic agent, buffering agent, soothing agent and the like for liquid preparations, and base, emulsifier, wetting agent, stabilizer, stabilizing agent, dispersing agent, plasticizer, pH adjuster, absorption promoter, gelling agent, antiseptic, filler, dissolving agent, solubilizing agent, suspending agent and the like for semisolid preparations. Where necessary, moreover, additives such as preservative, antioxidant, colorant, sweetening agent and the like may be used.

Examples of the "excipient" include lactose, sucrose, D-mannitol, D-sorbitol, cornstarch, dextrin, microcrystalline cellulose, crystalline cellulose, carmellose, carmellose calcium, sodium carboxymethyl starch, low-substituted hydroxypropylcellulose, gum arabic and the like.

Examples of the "disintegrant" include carmellose, carmellose calcium, carmellose sodium, sodium carboxymethyl starch, croscarmellose sodium, crospovidone, low-substituted hydroxypropylcellulose, hydroxypropylmethylcellulose, crystalline cellulose and the like.

Examples of the "binder" include hydroxypropylcellulose, hydroxypropylmethylcellulose, povidone, crystalline cellulose, sucrose, dextrin, starch, gelatin, carmellose sodium, gum arabic and the like.

Examples of the "fluidizer" include light anhydrous silicic acid, magnesium stearate and the like.

Examples of the "lubricant" include magnesium stearate, calcium stearate, talc and the like.

Examples of the "solvent" include purified water, ethanol, propylene glycol, macrogol, sesame oil, corn oil, olive oil and the like.

Examples of the "solubilizing agents" include propylene glycol, D-mannitol, benzyl benzoate, ethanol, triethanolamine, sodium carbonate, sodium citrate and the like.

Examples of the "suspending agent" include benzalkonium chloride, carmellose, hydroxypropylcellulose, propylene glycol, povidone, methylcellulose, glycerol monostearate and the like.

Examples of the "isotonic agent" include glucose, D-sorbitol, sodium chloride, D-mannitol and the like.

Examples of the "buffering agent" include sodium hydrogenphosphate, sodium acetate, sodium carbonate, sodium citrate and the like.

Examples of the "soothing agent" include benzyl alcohol and the like.

Examples of the "base" include water, animal and vegetable oils (olive oil, corn oil, peanut oil, sesame oil, castor oil etc.), lower alcohols (ethanol, propanol, propylene glycol, 1,3-butyleneglycol, phenol etc.), higher fatty acid and ester thereof, waxes, higher alcohol, polyhydric alcohol, hydrocarbons (white petrolatum, liquid paraffin, paraffin etc.), hydrophilic petrolatum, purified lanolin, absorption ointment, hydrolyzed lanolin, hydrophilic ointment, starch, pullulan, gum arabic, gum tragacanth, gelatin, dextran, cellulose derivative (methylcellulose, carboxymethylcellulose, hydroxyethylcellulose, hydroxypropylcellulose etc.), synthetic polymers (carboxyvinyl polymer, sodium polyacrylate, poly(vinyl alcohol), polyvinylpyrrolidone etc.), propylene glycol, macrogol (macrogol 200 - 600 etc.), and combinations of two or more kinds thereof.

Examples of the "preservative" include ethyl parahydroxybenzoate, chlorobutanol, benzyl alcohol, sodium dehydroacetate, sorbic acid and the like.

Examples of the "antioxidant" include sodium sulfite, ascorbic acid and the like.

Examples of the "colorant" include food colors (e.g., Food Color Red No. 2 or 3, Food Color yellow No. 4 or 5 etc.), β-carotene and the like.

Examples of the "sweetening agent" include saccharin sodium, dipotassium glycyrrhizinate, aspartame and the like.

The dose of the therapeutic or prophylactic agent of the present invention to mammals including human (e.g., human, mouse, rat, hamster, guinea pig, rabbit, cat, dog, swine, bovine, horse, sheep, monkey etc.) varies depending on the subject of administration, disease, symptom, dosage form, administration route and the like. For example, in the case of human, the dose for oral administration to an adult patient (body weight about 60 kg) is generally 0.1 mg - 1 g, preferably 30 mg - 900 mg, per day based on the active ingredient compound A. This amount can be administered in one to several portions per day, before meal, after meal or between meals. The dosing period is not particularly limited.

As a cause of CKD, for example, the diseases recited in Table 1 can be mentioned ("Chronic Kidney Disease" Merck Manual Professional Version (Online Edition), Anna Malkina, updated October 2018, URL: https://www.merckmanuals.com/en-pr/professional/genitourinary-disorders/chronic-kidney-disease/chronic-kidney-disease) .

**[Table 1]**

| cause | example |
|---|---|
| chronic renal tubulointerstitial nephropathy | cause of chronic renal tubule interstitial nephritis |
| glomerulopathy (primary) | focal glomerulosclerosis idiopathic crescentic glomerulonephritis IgA nephropathy membranoproliferative glomerulonephritis membranous nephropathy |
| glomerulopathy associated with systemic disease | amyloidosis diabetes anti-GBM antibody disease (Goodpasture's syndrome) granulomatosis with polyangiitis hemolytic-uremic syndrome mixed cryoglobulinemia postinfectious glomerulonephritis systemic lupus erythematosus |
| hereditary nephropathy | autosomal dominant tubulointerstitial kidney disease (medullary cystic kidney) hereditary nephritis (Alport's syndrome) nail-patella syndrome polycystic kidney |
| hypertension | hypertensive nephrosclerosis |
| obstructive urethral disease | prostatomegaly posterior urethral valve retroperitoneal fibrosis ureteral obstruction (congenital, calculus, cancer) vesicoureteral reflux |
| renal macroangiopathy (vascular disorder of renal artery and vein) | renal artery stenosis due to arteriosclerosis or fibromuscular dysplasia |

CKD caused by diabetes includes diabetic nephropathy and diabetic kidney disease. In some embodiments, CKD is a CKD not including diabetic nephropathy, preferably a CKD not caused by diabetes (primary disease).

In some other embodiments, CKD is CKD caused by one or more diseases selected from the group consisting of
(1) chronic renal tubulointerstitial nephropathy,
(2)
   (A) focal glomerulosclerosis,
   (B) idiopathic crescentic glomerulonephritis,
   (C) IgA nephropathy,
   (D) membranoproliferative glomerulonephritis,
   (E) membranous nephropathy,
(3)
   (A) amyloidosis,
   (B) anti-GBM antibody disease (Goodpasture's syndrome),
   (C) granulomatosis with polyangiitis,
   (D) hemolytic-uremic syndrome,
   (E) mixed cryoglobulinemia,
   (F) postinfectious glomerulonephritis,
   (G) systemic lupus erythematosus,
(4)
   (A) autosomal dominant tubulointerstitial kidney disease (medullary cystic kidney),
   (B) hereditary nephritis (Alport's syndrome),
   (C) nail-patella syndrome,
   (D) polycystic kidney,
(5) hypertension,
(6)
   (A) prostatomegaly
   (B) posterior urethral valve
   (C) retroperitoneal fibrosis
   (D) ureteral obstruction (congenital, calculus, cancer),
   (E) vesicoureteral reflux, and
(7) renal macroangiopathy.

Furthermore, in some other embodiments, CKD is a CKD caused by diabetes, preferably diabetic kidney disease (DKD) or diabetic nephropathy. Currently, CKD patients with diabetes are being treated by blood pressure management with ARB or ACE inhibitor and the like (rather than diabetic drugs) (Kidney Int Suppl (2012) Vol.2, pp.363-369; Am J Kidney Dis (2013) Vol.62, pp.201-213; Nephol Dial Transplant (2014) Vol.29, pp.490-496). While such blood pressure management is no doubt beneficial to those patient groups, it cannot be said that the blood pressure lowering strategy fulfills all of the therapeutic needs (Lancet (2015) Vol.385, pp.2047-2056). As specifically explained in the following Experimental Examples, compound A or a pharmaceutically acceptable salt thereof, or a monohydrate of compound A surprisingly reduces the severity of the disease, and therefore provides another option for the prophylaxis or treatment of DKD or diabetic nephropathy.

In the present specification, the "treatment" means, (1) improvement of renal function, renal disorder, or the symptoms of CKD, (2) prevention or delay of decreased renal function, progression of renal disorder, or aggravation of the symptoms of CKD, or (3) prevention of decrease of renal function, progression of renal disorder, or recurrence of the symptoms of CKD.

In the present specification, "prophylaxis" means (4) suppression of decrease in GFR or eGFR to less than 60 mL/min/1.73 m², increase in urinary protein value to 0.15 g/gCr or more, or increase in urinary albumin value to not less than 30 mg/gCr, or prevention of continuation of the state of (4) for 3 months or more.

In the present specification, the "improvement of renal function" means improvement, or discontinuation of decrease, or delay of decrease in renal function. The renal function can be evaluated by changes in the parameters such as GFR, eGFR, creatinine clearance, urinary serum creatinine value, blood urea nitrogen value, urinary protein value, urinary albumin value and the like.

The "improvement of renal function" is preferably an improvement of renal function of CKD patients.

The improvement of GFR, eGFR, serum creatinine value, blood urea nitrogen value, creatinine clearance, urinary protein value or urinary albumin value is an improvement of one or more parameters selected from GFR, eGFR, serum creatinine value, blood urea nitrogen value, creatinine clearance, urinary protein value and urinary albumin value, preferably an improvement of these parameters in CKD patients.

In the present specification, the "improvement of GFR" means increase, or discontinuation of decrease, or delay of decrease in GFR. In one embodiment, the "improvement of GFR" is improvement of GFR in patients with GFR of 90 ml/min or less.

In the present specification, the "improvement of eGFR" means increase, or discontinuation of decrease, or delay of decrease in eGFR. In one embodiment, the "improvement of eGFR" is improvement of eGFR in patients with eGFR of 90 ml/min/1.73 m² or less.

In the present specification, the "improvement of creatinine clearance" means increase, or discontinuation of decrease, or delay of decrease in creatinine clearance. In one embodiment, the "improvement of creatinine clearance" is improvement of creatinine clearance in patients with creatinine clearance of 100 mL/min or less.

In the present specification, the "improvement of serum creatinine value" means decrease, or discontinuation of increase, or delay of increase in the serum creatinine value. In one embodiment, the "improvement of serum creatinine value" is improvement of serum creatinine value in patients with a serum creatinine value of 1.2 mg/dl or more.

In the present specification, the "improvement of blood urea nitrogen value" means decrease, or discontinuation of increase, or delay of increase in the blood urea nitrogen value. In one embodiment, the "improvement of blood urea nitrogen value" is improvement of the blood urea nitrogen value in patients with a blood urea nitrogen value of 20 mg/dl or more.

In the present specification, the "improvement of urinary protein value" means decrease, or discontinuation of increase, or delay of increase in the urinary protein value. In one embodiment, the "improvement of urinary protein value" is improvement of the urinary protein value in patients with a urinary protein value of 0.15 g/gCr or more.

In the present specification, the "improvement of urinary albumin value" means decrease, or discontinuation of increase, or delay of increase in the urinary albumin value. In one embodiment, the "improvement of urinary albumin value" is improvement of the urinary albumin value in patients with a urinary albumin value of 30 mg/gCr or more.

An index of the severity of CKD is, for example, GFR section. The compound A or a pharmaceutically acceptable salt thereof, or a monohydrate of compound A can be used for inhibiting or delaying the progression of the stage by GFR section. For example, compound A or a pharmaceutically acceptable salt thereof, or a monohydrate of compound A can be used for inhibiting or delaying the progression from stage G1 to G2, G2 to G3a, G3a to G3b, G3b to G4, and G4 to G5.

The compound A or a pharmaceutically acceptable salt thereof, or a monohydrate of compound A can be used for the prophylaxis or treatment of CKD since it improves disordered changes in glomerulus, renal tubule or kidney blood vessel.

More specifically, compound A or a pharmaceutically acceptable salt thereof, or a monohydrate of compound A improves hardening, fibrosis, disintegration, hypertrophy, or exudative changes of glomerulus, improves expansion of renal tubule, or improves thickening of arteriole wall or fibrotic and necrotic changes of arterial wall in kidney blood vessels.

The compound A or a pharmaceutically acceptable salt thereof, or a monohydrate of compound A can be used for the prophylaxis or treatment of the symptoms of CKD such as nocturia, edema, anemia, fatigue, shortness of breath and the like.

The pharmaceutical composition of the present invention can be used in combination with one or a plurality of other medicaments (hereinafter to be also referred to as a concomitant drug) according to a method generally employed in the pharmaceutical field (hereinafter to be referred to as combined use).

The administration timing of medicaments and concomitant drugs containing compound A or a pharmaceutically acceptable salt thereof, or a monohydrate of compound A is not limited, and they may be administered to an administration subject as combination preparation, or the both preparations may be administered simultaneously or at given intervals.

In addition, the pharmaceutical composition of the present invention and a concomitant drug may be used as a medicament in the form of a kit. The dose of the concomitant drug is similar to the clinically-employed dose and can be appropriately selected according to the subject of administration, disease, symptom, dosage form, administration route, administration time, combination and the like. The administration form of the concomitant drug is not particularly limited, and it only needs to be combined with a medicament containing compound A or a pharmaceutically acceptable salt thereof, or a monohydrate of compound A.

Examples of the concomitant drug include angiotensin converting enzyme (ACE) inhibitor, angiotensin II receptor antagonists (ARB), calcium antagonist, diuretic, alpha-blocking agent, beta-blocking agent and the like can be mentioned.

Examples of the "angiotensin converting enzyme inhibitor" include, but are not limited to, benazepril, captopril, enalapril, fosinopril, lisinopril, moexipril, perindopril, quinapril, ramipril, trandolapril, imidapril, alacepril, delapril, cilazapril, temocapril and the like. Angiotensin converting enzyme inhibitors can also be used in the form of a pharmaceutically acceptable salt thereof or a pharmaceutically acceptable prodrug.

Examples of the "angiotensin II receptor antagonist" include, but are not limited to, candesartan, eprosartan, irbesartan, telmisartan, valsartan, losartan, olmesartan and the like. Angiotensin II receptor antagonists can also be used in the form of a pharmaceutically acceptable salt thereof or a pharmaceutically acceptable prodrug.

Examples of the "calcium antagonist" include, but are not limited to, amlodipine, nifedipine, nicardipine, diltiazem, manidipine, benidipine, nilvadipine, nitrendipine, nisoldipine, barnidipine and the like. Calcium antagonists can also be used in the form of a pharmaceutically acceptable salt thereof or a pharmaceutically acceptable prodrug.

Examples of the "diuretic" include, but are not limited to, spironolactone, hydrochlorothiazide, furosemide, triamterene and the like. Diuretics can also be used in the form of a pharmaceutically acceptable salt thereof or a pharmaceutically acceptable prodrug.

Examples of the "alpha-blocking agent" include, but are not limited to, doxazosin, prazosin, bunazosin, terazosin, urapidil and the like. Alpha-blocking agents can also be used in the form of a pharmaceutically acceptable salt thereof or a pharmaceutically acceptable prodrug.

Examples of the "beta-blocking agent" include, but are not limited to, metoprolol, atenolol, bisoprolol, arotinolol, celiprolol, betaxolol and the like. Beta-blocking agents can also be used in the form of a pharmaceutically acceptable salt thereof or a pharmaceutically acceptable prodrug.

One embodiment of the present invention is a method for the prophylaxis or treatment of a chronic kidney disease, including administering a therapeutically effective amount of compound A or a pharmaceutically acceptable salt thereof, or a monohydrate of compound A to a mammal. The definitions and the like are as previously described.

In the present specification, the "effective amount" means, for example, the amount of a medicament or drug that induces a biological or medical response in a tissue, system, animal or human. The "therapeutically effective amount" means any amount that results in a treatment, cure, prevention or improvement of a disease, disorder or side effect, or reduces the rate of progression of the disease, as compared to the corresponding subject who did not receive such amount.

One embodiment of the present invention is a pharmaceutical composition for the prophylaxis or treatment of a chronic kidney disease, containing compound A or a pharmaceutically acceptable salt thereof, or a monohydrate of compound A. The definitions and the like are as previously described.

One embodiment of the present invention is use of compound A or a pharmaceutically acceptable salt thereof, or a monohydrate of compound A in producing a therapeutic or prophylactic agent for a chronic kidney disease. The definitions and the like are as previously described.

One embodiment of the present invention is compound A or a pharmaceutically acceptable salt thereof, or a monohydrate of compound A for use in the prophylaxis or treatment of a chronic kidney disease. The definitions and the like are as previously described.

One embodiment of the present invention is a therapeutic or prophylactic agent for a chronic kidney disease (CKD), containing a fused heterocyclic compound selected from the following formulas:

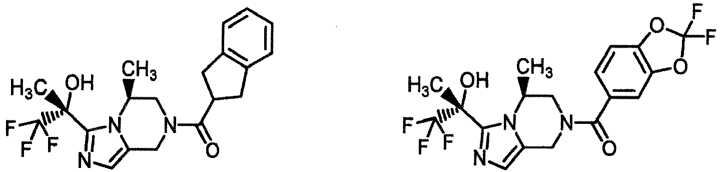

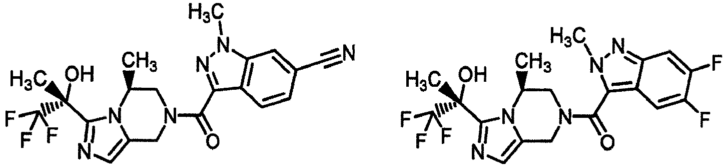

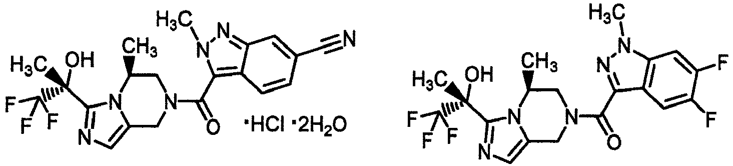

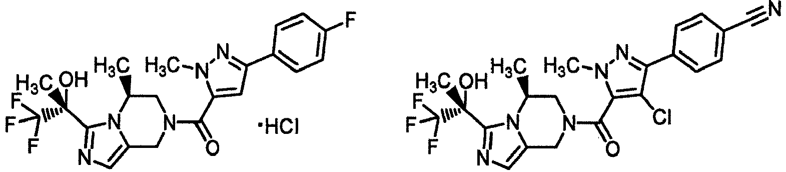

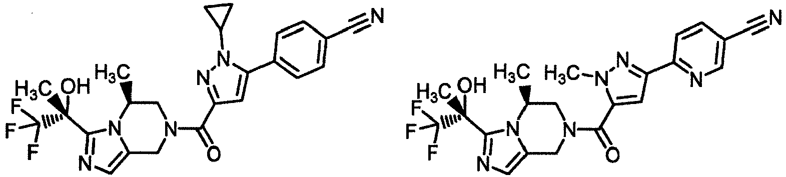

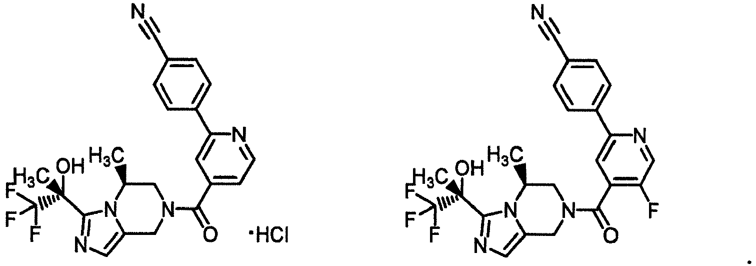

The method of use, the effective amount of the fused heterocyclic compound, the formulation method, and the like are the same as or similar to those in the aforementioned embodiments using compound A or a pharmaceutically acceptable salt thereof.

### [Example]

The present invention is specifically explained in the following by referring to Examples. The present invention is not limited to these Examples.

As a Formulation Example of the present invention, the following preparation can be mentioned. However, the present invention is not limited by these Formulation Examples.

### Formulation Example 1: production of capsule

1) monohydrate of compound A 50 mg
2) microcrystalline cellulose 10 mg
3) lactose 19 mg
4) magnesium stearate 1 mg
   1), 2), 3) and 4) are mixed and filled in a gelatin capsule.

### Formulation Example 2: production of tablet

1) monohydrate of compound A 50 g
2) lactose 50 g
3) cornstarch 15 g
4) carmellose calcium 44 g
5) magnesium stearate 1 g

The total amount of 1), 2), 3) and 30 g of 4) are kneaded with water, vacuum dried, and sieved. The sieved powder is mixed with 14 g of 4) and 1 g of 5), and the mixture is punched by a tableting machine. In this way, 1000 tablets each containing 50 mg of the monohydrate of compound A per tablet are obtained.

### Experimental Example 1: Suppressive effects of compound A on urinary protein excretion and renal disorder by oral administration of monohydrate of compound A in saline-loaded SDT-fatty rat model

In saline-loaded Spontaneously Diabetic Torii fatty (SDT-fatty) rats, suppressive effects of compound A on urinary protein excretion and renal disorder were evaluated. The evaluation was performed by reference to non-patent document 1. As the experiment animal, 6-week-old male SDT-fatty rats (CLEA Japan, Inc.) were used. A normal powder feed (CRF-1 powder, Oriental Yeast Co., ltd.) containing 0.005 w/w% of a monohydrate of compound A as a free form of compound A was prepared.

1 w/v% of sodium chloride solution (saline)
was supplied to the control group (Vehicle) and the compound A administration group (compound A). The group supplied with water free of sodium chloride was used as the pathology control group (Normal). Compound A was orally administered to the compound A administered group for 14 weeks by feeding the above-mentioned feed containing compound A at the same time as the supply of saline. The control group and the pathology control group were fed with the above-mentioned normal powder feed without containing compound A. Urine was collected for 6 hrs (10 weeks after the start of administration) and the kidney was removed (14 weeks after the start of administration).

For urine, after measuring the urine volume, the protein concentration in the urine was measured by the absorbance at wavelength 590 nm using a total protein quantification kit (TONEIN-TP, Otsuka Pharmaceutical Co., Ltd.). Amount of the urinary protein excretion (mg/6 hours) was calculated from the urine volume and protein concentration.

For kidney, tissue was fixed with a 10% neutral buffered formalin solution (Wako Pure Chemical Industries, Ltd.), and the tissue section was stained with Hematoxylin Eosin (HE).

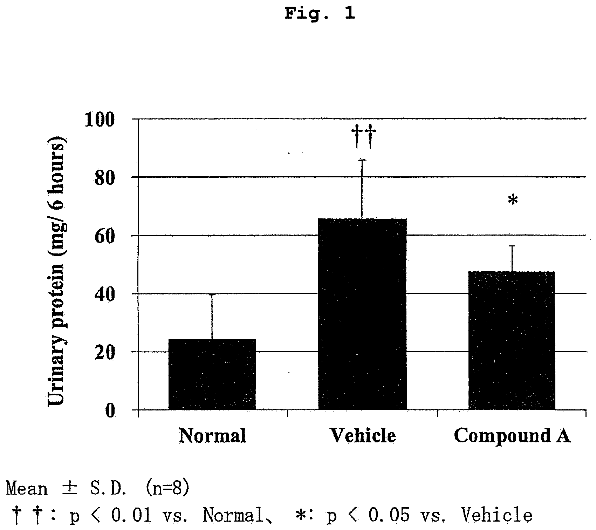

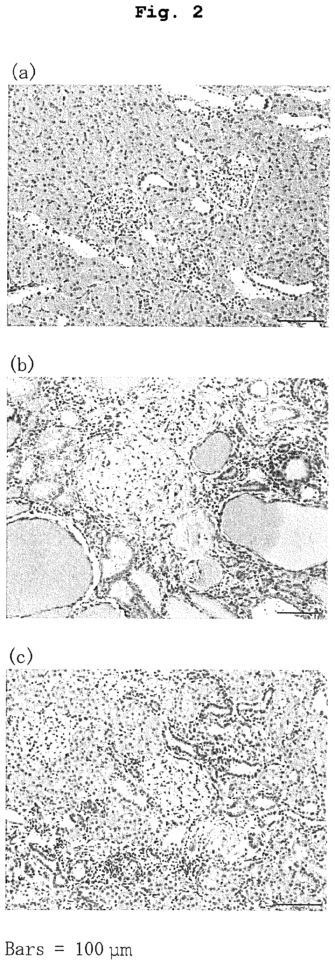

The mean and standard deviation of urinary protein excretion for each group were calculated, and the results thereof are shown in Fig. 1. Representative examples of the kidney tissue images are shown in Fig. 2.

### Experimental Example 2: Suppressive effects of compound A on urinary protein excretion and renal disorder by oral administration of monohydrate of compound A in 5/6 nephrectomized rat model

In 5/6 nephrectomized rats, suppressive effects of compound A on urinary protein excretion and renal disorder were evaluated. The evaluation was performed by reference to non-patent document 2. As the experiment animal, male Wistar rats (Japan SLC, Inc.) in which 60% of the right kidney was removed at 7 weeks of age and the left kidney was completely removed at 8 weeks of age were used. A group that was treated similarly except for nephrectomy was used as a Sham group (Sham). A normal powder feed (CRF-1 powder, Oriental Yeast Co., ltd.) containing 0.0218 to 0.0317 w/w% of a monohydrate of compound A as a free form of compound A was prepared.

Compound A was orally administered to the compound A administration group for 10 weeks by feeding the above-mentioned feed containing compound A from 21 weeks of age. The control group (Vehicle) and Sham group were fed with the above-mentioned normal powder feed without containing compound A. Urine was collected for 24 hrs (5 weeks after the start of administration) and the kidney was removed (10 weeks after the start of administration).

For urine, after measuring the urine volume, the protein concentration was measured by the absorbance at wavelength 590 nm using a total protein quantification kit (TONEIN-TP, Otsuka Pharmaceutical Co., Ltd.). Amount of the urinary protein excretion (mg/day) was calculated from the urine volume and protein concentration.

For kidney, tissue was fixed with a 10% neutral buffered formalin solution (Wako Pure Chemical Industries, Ltd.), and the tissue section was stained with Hematoxylin Eosin (HE).

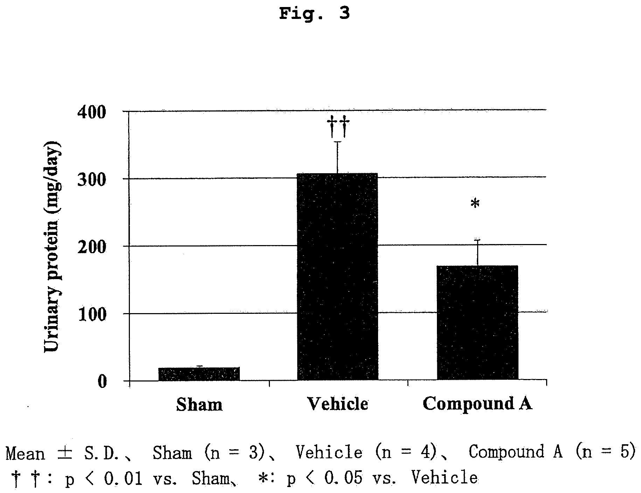

The mean and standard deviation of amount of urinary protein excretion for each group were calculated, and the results thereof are shown in Fig. 3. Disorders at each site of kidney tissue were evaluated by score and are shown in Table 2.

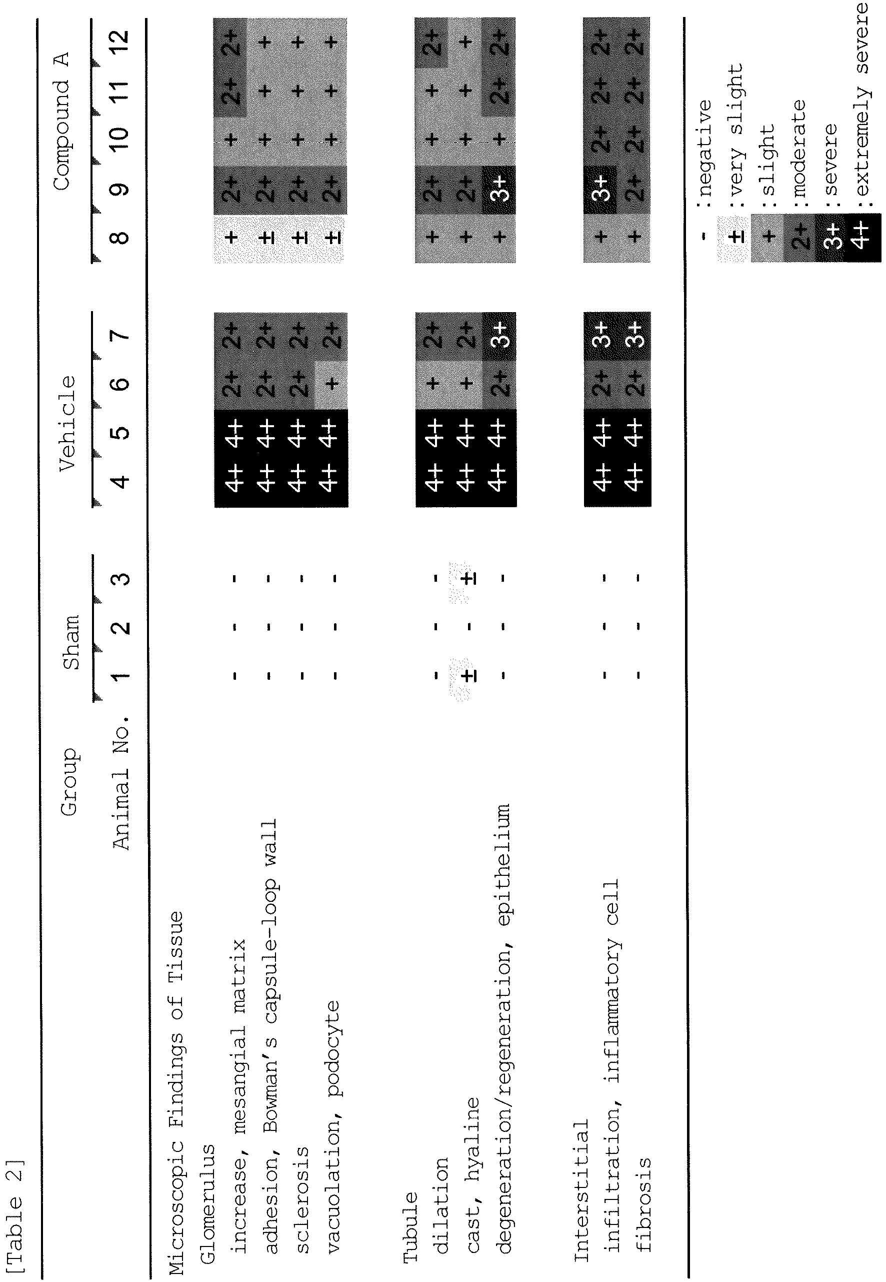

### Reference Example 1: Suppressive effects of compound A on urinary protein excretion and renal disorder by oral administration of fused heterocyclic compound in saline-loaded SDT-fatty rat model

As used herein, the fused heterocyclic compound is a compound selected from the following formulas:

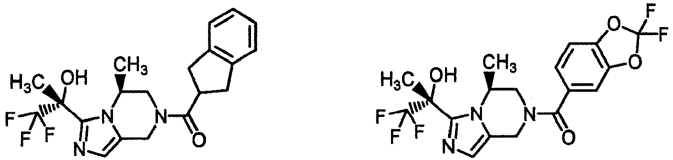

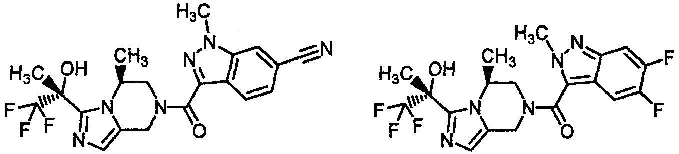

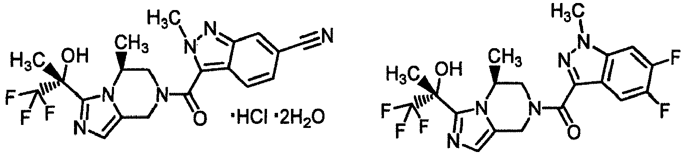

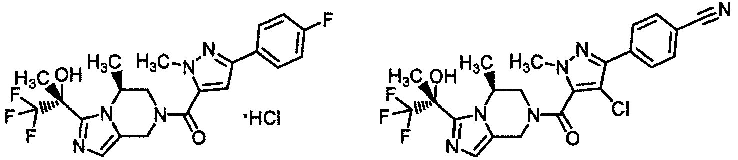

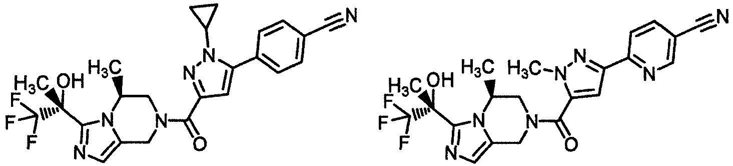

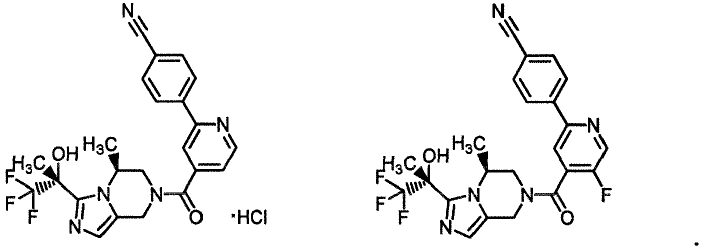

In saline-loaded SDT-fatty rats, suppressive effects of the aforementioned fused heterocyclic compound on urinary protein excretion and renal disorder are evaluated. As the experiment animal, 6-week-old male SDT-fatty rats (CLEA Japan, Inc.) are used. The fused heterocyclic compound can be each obtained by the method described in patent document 3 (WO 2019/151274). A normal powder feed (CRF-1 powder, Oriental Yeast Co., ltd.) containing each fused heterocyclic compound is prepared.

1 w/v% of saline is supplied to the control group (Vehicle) and the fused heterocyclic compound administration group. The group supplied with water free of sodium chloride is used as the pathology control group (Normal). The fused heterocyclic compound is orally administered to the fused heterocyclic compound administered group for 14 weeks by feeding the above-mentioned feed containing the aforementioned fused heterocyclic compound at the same time as the supply of saline. The control group and the pathology control group are fed with the above-mentioned normal powder feed without containing the aforementioned fused heterocyclic compound. Urine is collected for 6 hrs (10 weeks after the start of administration) and the kidney is removed (14 weeks after the start of administration).

For urine, after measuring the urine volume, the protein concentration in the urine is measured by the absorbance at wavelength 590 nm using a total protein quantification kit (TONEIN-TP, Otsuka Pharmaceutical Co., Ltd.). Amount of the urinary protein excretion (mg/6 hours) is calculated from the urine volume and protein concentration.

For kidney, tissue is fixed with a 10% neutral buffered formalin solution (Wako Pure Chemical Industries, Ltd.), and the tissue section is stained with Hematoxylin Eosin (HE).

The mean and standard deviation of urinary protein excretion for each group are calculated.

### Reference Example 2: Suppressive effects of fused heterocyclic compound on urinary protein excretion and renal disorder by oral administration of fused heterocyclic compound in 5/6 nephrectomized rat model

In 5/6 nephrectomized rats, suppressive effects of the aforementioned fused heterocyclic compound on urinary protein excretion and renal disorder are evaluated. As the experimental animal, male Wistar rats (Japan SLC, Inc.) in which 60% of the right kidney was removed at 7 weeks of age and the left kidney was completely removed at 8 weeks of age are used. A group that was treated similarly except for nephrectomy is used as a Sham group (Sham). A normal powder feed (CRF-1 powder, Oriental Yeast Co., ltd.) containing each fused heterocyclic compound is prepared.

The aforementioned fused heterocyclic compound is orally administered to the aforementioned fused heterocyclic compound administration group for 10 weeks by feeding the above-mentioned feed containing the aforementioned fused heterocyclic compound from 21 weeks of age. The control group (Vehicle) and Sham group are fed with the above-mentioned normal powder feed without containing the aforementioned fused heterocyclic compound. Urine is collected for 24 hrs (5 weeks after the start of administration) and the kidney is removed (10 weeks after the start of administration).

For urine, after measuring the urine volume, the protein concentration is measured by the absorbance at wavelength 590 nm using a total protein quantification kit (TONEIN-TP, Otsuka Pharmaceutical Co., Ltd.). Amount of the urinary protein excretion (mg/day) is calculated from the urine volume and protein concentration.

For kidney, tissue is fixed with a 10% neutral buffered formalin solution (Wako Pure Chemical Industries, Ltd.), and the tissue section is stained with Hematoxylin Eosin (HE).

The mean and standard deviation of amount of urinary protein excretion for each group are calculated.

### [Industrial Applicability]

The present invention provides a novel pharmaceutical use of compound A or a pharmaceutically acceptable salt thereof, or a monohydrate of compound A for CKD as the target disease.

The present invention is based on patent application No. 2018-169632, the contents of which are encompassed in full herein.

## Claims

1. A therapeutic or prophylactic agent for a chronic kidney disease, comprising a compound represented by the following chemical structural formula: 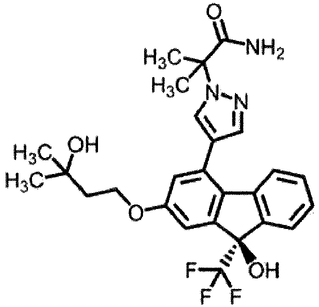 or a pharmaceutically acceptable salt thereof, or a compound represented by the following chemical structural formula:

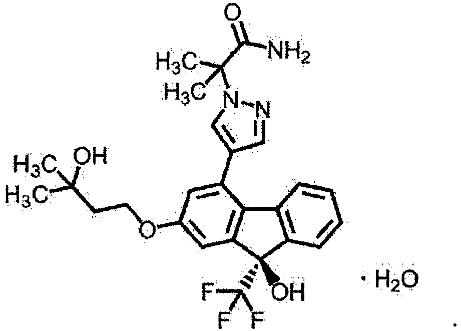

2. A renal function improving agent comprising a compound represented by the following chemical structural formula: 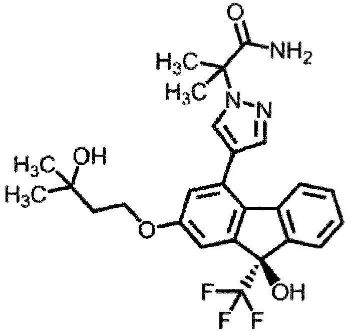 or a pharmaceutically acceptable salt thereof, or a compound represented by the following chemical structural formula:

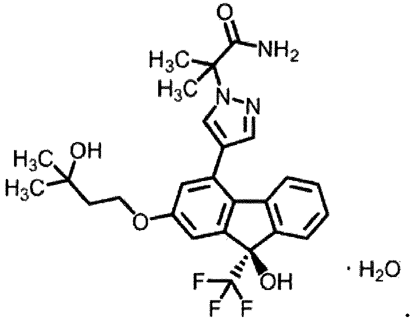

3. An agent for improving one or more parameters selected from GFR, eGFR, serum creatinine value, blood urea nitrogen value, creatinine clearance, urinary protein value and urinary albumin value, comprising a compound represented by the following chemical structural formula: 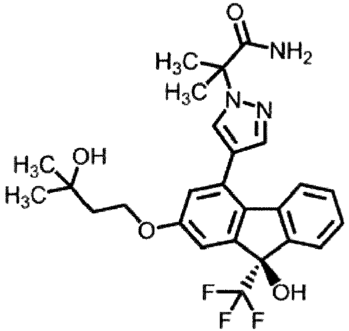 or a pharmaceutically acceptable salt thereof, or a compound represented by the following chemical structural formula:

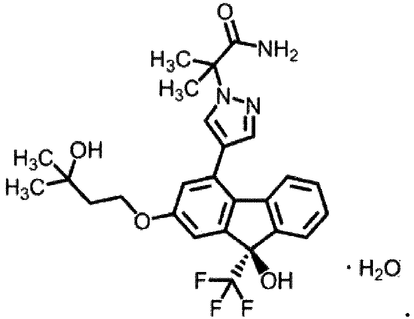

4. The agent according to any one of claims 1 to 3, comprising a compound represented by the following chemical structural formula:

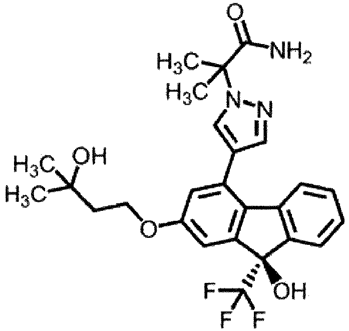

5. The agent according to any one of claims 1 to 3, comprising a compound represented by the following chemical structural formula:
